# EUROPEAN PATENT APPLICATION

(11) **EP 4 467 168 A1**
(43) Date of publication of application: **27.11.2024**
(21) Application number: 23743398.2
(22) Date of filing: 09.01.2023
(51) Int. Cl.: A61L 27/12, A61L 27/54, A61L 27/04

(54) **APATITE FOR FILLER AND FILLER COMPOSITION COMPRISING SAME**

(30) Priority: 18.01.2022 KR 20220007333
(71) Applicant: Korea Institute of Science and Technology, Seoul 02792 (KR)
(72) Inventor: JEON, Hojeong, Seoul 02792 (KR); LEE, Kyungwoo, Seoul 02792 (KR); SONG, Sangmin, Seoul 02792 (KR); UM, Seung-Hoon, Seoul 02792 (KR); HAN, Hyung-Seop, Seoul 02792 (KR); LEE, Hyojin, Seoul 02792 (KR); LEE, Wonryung, Seoul 02792 (KR); OK, Myoung-Ryul, Seoul 02792 (KR); KIM, Yu Chan, Seoul 02792 (KR)
(74) Representative: Gulde & Partner
(86) International application number: PCT/KR2023/000383
(87) International publication number: WO 2023/140549

(57) **Abstract**

Provided are an apatite for a filler and a filler composition comprising an apatite that contains metal, wherein the metal entirely or partially substitutes the calcium of the apatite.

## Description

### [Technical field]

The present invention relates to an apatite for a filler and a filler composition including the same, and more specifically, to an apatite for a filler, which promotes collagen secretion inside the gums while increasing the duration of the filler and may be expected to have an antibacterial effect, and a filler composition including the same.

### [National research and development project that supported this invention]

- Unique project number: 1711145260
- Project number: 2020R1A2C2010413
- Name of ministry: Ministry of Science and ICT
- Project management (professional) organization name: National Research Foundation of Korea
- Research project name: Individual basic research (Ministry of Science and ICT) (R&D)
- Research project title: Development of laser single process technology for highspeed/low-cost apatite preparation and coating
- Name of organization performing the project: Korea Institute of Science and Technology

### [Background Art]

In general, the oral environment is always wet due to saliva and food, unlike other facial tissues. In saliva, various microorganisms such as bacteria and fungi are present due to food intake, substances such as food residues, tartar, plaque and tongue coating, inflammatory cells, and minerals are present, and saliva is a special part of the body where various dental materials (titanium, gold alloys, other metal alloys, amalgams, resin, zirconia, porcelain, and the like) from different dental treatments for each patient coexist.

In addition, unlike other facial areas, oral soft tissue has unique properties that distinguish it from other human tissues in order to resist stress caused by various movements such as speech, mastication, and swallowing.

Meanwhile, hyaluronic acid, a filler composition mainly used for wound repair or wrinkle improvement in the facial area, is a linear polymer polysaccharide in which β-D-N-acetylglucosamine and β-D-glucuronic acid are alternately bonded. In addition to being distributed in connective tissues such as the subcutaneous tissue and cartilage tissue of mammals, hyaluronic acid is known to present in the vitreous body of the eye, the umbilical cord, or the like, and also in the capillaries of streptococci and bacilli. Common methods for obtaining hyaluronic acid include a method of extracting it from chicken combs, umbilical cords or the like and a method of cultivating streptococci and bacilli, extracting it from the culture and then purifying it.

Natural hyaluronic acid, which has excellent biocompatibility, has no cross-species specificity and no tissue or organ specificity, improves the skin's moisturizing ability, maintains skin elasticity, and reduces damage to the lower layer of the skin when skin is damaged, and also acts like a lubricant to help collagen, a major component of skin, move smoothly between cells.

However, when natural hyaluronic acid is used as is, not only does it have poor mechanical properties, but it is also easily decomposed and removed by an enzyme called hyaluronidase present in the body, leading to limitations in various applications. In order to compensate for these shortcomings of natural hyaluronic acid, many studies are being conducted to form hydrogels through chemical modification or crosslinking using various crosslinking agents.

Hydrogel formation through chemical modification and crosslinking of hyaluronic acid is generally achieved through the alcohol group and carboxylic acid group present in the main chain of hyaluronic acid.

The carboxylic acid group of the hyaluronic acid main chain is mainly chemically modified by esterification (D. Campoccia et al., Biomaterials, 19, 1998, 2101-2127), and the crosslinking reaction for hydrogel formation has been studied using dihydrazide (K. P. Vercruysse et al., Bioconjug. Chem., 8, 1997, 686-694), dialdehyde (Y. Luo et al., J. Control. Release, 69, 2000, 169-184), or disulfide (X. Z. Shu et al., Biomacromolecules, 3, 2002, 1304-1311).

In addition, research was conducted to prepare hydrogels through photocrosslinking by introducing methacrylamide into the carboxylic acid group (Y. D. Park et al., Biomaterials, 24, 2003, 893-900). On the other hand, research was conducted on the alcohol group of the hyaluronic acid main chain using divinyl sulfone (A. Ramamurthi et al., J. Biomed. Master Res., 60, 2002, 195-205) or diglycidyl ether (T. Segura et al., Biomaterials, 26, 2005, 359-371).

Meanwhile, from the late 1980s to the 1990s, research on hydrogel particles has been applied to fields such as embolization, enzyme immobilization, and drug delivery by converting polymer materials into micro-sized particles and chemically modifying the surface and interior. Entering the 2000s, along with the development of nanotechnology, research on the preparation and application of nano-sized particles using water-soluble polymers is being conducted.

To date, most research on hydrogel particles uses biodegradable polymers for the purpose of application in the fields of new bio drugs and bio organs, and is focused on manufacturing injectable structures for non-invasive procedures.

However, these conventional filler compositions that were mainly applied to the facial area had problems in that they could not solve problems caused by an insufficient volume or area of the gingiva in the patient's mouth, such as non-aesthetic prosthesis and discomfort due to gingival recession or lack of soft tissue during various prosthetic treatments, hyperesthesia due to tooth root exposure, non-aesthetic gingival coloring, non-aesthetic prosthesis due to gingival recession between teeth, and the like.

That is, dentally, to date, when space remains below the prosthesis (dentures, bridges, etc.) over time after fabrication of the dental prosthesis due to alveolar bone resorption and gingival recession, because the patient's discomfort (pain, bad odor due to food retention, etc.) is very high, it is common to remanufacture and repair prosthetics before using them. In addition, when a black triangle occurs in the space between two teeth due to gingival recession, a very sophisticated periodontal surgery is performed in which gingival tissue from the palate or other areas is transplanted and fixed to the receding area, and even after a healing period of 4-8 weeks, the success rate is not high, so an experienced doctor must perform the procedure, but in reality, even when a dental prosthesis was made to close an empty space or a gum-colored prosthesis was made, it was very unaesthetic and patients were highly dissatisfied.

That is, in the past, because an appropriate dental filler composition was not developed, there were many problems in that the space remains between implants or between teeth and implants due to lack of soft tissue in the treatment of gums during implant surgery, causing non-aesthetic problems.

### [Related Art Documents]

Korea Patent No. 10-0898491
Korea Patent No. 10-0959405
Korea Patent Publication No. 10-2021-0123581

### [Disclosure]

### [Technical Problem]

Therefore, the problem to be solved by the present invention is to provide a new oral restorative filler that may restore tissue in the space between implants or between teeth and implants due to lack of soft tissue in the treatment of gums during implant surgery, and that may simultaneously suppress inflammation occurring within the oral cavity.

### [Technical Solution]

In order to solve the above problems, the present invention provides an apatite for a filler including an apatite that contains metal, wherein the metal entirely or partially replaces the calcium of the apatite.

In one embodiment of the present invention, the metal may be at least one selected from the group consisting of magnesium (Mg), zinc (Zn), and strontium (Sr), and the metal of the apatite may replace the calcium of the apatite in a solution using a laser.

In one embodiment of the present invention, the apatite may have a quadrangular shape.

The present invention also provides a filler composition including the above-described apatite, and hyaluronic acid.

In one embodiment of the present invention, the metal may be eluted in the form of ions in vivo, and the filler composition may be for oral tissue.

In one embodiment of the present invention, the filler composition may have antibacterial properties as it is eluted in the form of ions in vivo.

In one embodiment of the present invention, in the apatite containing the metal, the metal may be present in an amount of 0.05 to 20% by weight of the total apatite, and the apatite may be present in an amount of 1 to 50% by weight of the composition.

### [Advantageous Effects]

The present invention provides a filler optimized for periodontal tissue restoration by combining hydroxyapatite and metal ion-substituted apatite with hyaluronic acid, which is widely used as an existing filler material, and the filler can promote collagen secretion inside the gums while increasing the duration of the filler, and can be expected to have an antibacterial effect.

### [Brief Description of Drawings]

FIG. 1 is an SEM image showing the morphology at low and high amounts of metal when Mg, Sr, and Zn are substituted.
FIG. 2 is an EDS image showing the morphology at low and high amounts of metal when Mg, Sr, and Zn are substituted.
FIG. 3 shows the results of SEM EDX analysis measuring the magnesium content of the composite when synthesized while increasing the amount of Mg added.
FIG. 4 shows the results of an ion elution amount experiment.
FIG. 5 shows an image of the shape of osteoblasts attached after cell culture.
FIG. 6 shows an optical image of a filler composition containing metal-substituted apatite (MgAp) mixed with hyaluronic acid hydrogel being discharged from a syringe, and an SEM image showing the morphology of the composition.
FIG. 7 shows the results of measuring viscoelasticity changes and viscosity rheological properties of the composition, and optical images by mass percentage percent concentration (w/w %) of metal-substituted apatite of compositions used for measurement.
FIG. 8 shows a graph measuring the swelling ratio of the composition.
FIG. 9 shows the results of an experiment on ion elution amount over time using the composition in phosphate buffered saline (PBS).
FIG. 10 shows the results of a proliferation test of gingival fibroblasts (HGF-1) for the composition.
FIG. 11 shows RT-PCR results for confirming the increase in expression of collagen type I alpha 1 (Col1a1) in gingival fibroblasts (HGF-1) according to time-dependent eluate treatment of substituted apatite at different concentrations.

### [Modes of the Invention]

Hereinafter, specific embodiments of the present invention will be described with reference to the drawings. However, this is only an example and the present invention is not limited thereto.

In the description of the present invention, when it is determined that a specific description of a known function or configuration may unnecessarily obscure the gist of the present invention, the detailed description thereof will be omitted. Also, the following terms are terms defined in consideration of functions in the present invention, which may vary according to the user, the intent or practice of the operator, etc. Therefore, the definition should be made on the basis of the contents throughout this specification.

The technical spirit of the present invention is determined by the claims, and the following examples are merely a means to efficiently explain the technical idea of the present invention to those skilled in the art in the technical field to which the present invention pertains.

Hydroxyapatite is a basic component of hard tissue in the human body and is also utilized as a bone tissue transplant or bone regeneration material. Hydroxyapatite has the chemical structure of Ca₁₀(PO₄)₆(OH)₂, and hydroxyapatite in human tooth enamel is mainly distributed in the outermost enamel, which is approximately 1-2 mm thick. It is known that such hydroxyapatite may exhibit a remineralization effect and thus has the function of directly filling micropores in demineralized enamel.

The present invention provides an oral filler prepared by replacing all or part of hydroxyapatite with a biodegradable metal and mixing it with hyaluronic acid. Hereinafter, according to the present invention, apatite containing a metal that elutes ions in vivo is referred to as "metal-substituted apatite."

The present invention will be described in more detail below through preferred Examples and Experimental Examples. However, the scope of the present invention is not limited by the following Examples and Experimental Examples.

### Example

### Synthesis of metal-substituted apatite

Metal-substituted apatite powder according to one embodiment of the present invention was prepared according to the laser hydrothermal synthesis method disclosed in Korean Patent Publication No. 10-2021-0123581. As metals, magnesium (Mg), zinc (Zn), and strontium (Sr), which are suitable for use in vivo and may elute metal ions, were used.

### Preparation of filler composition

A filler composition was prepared by mixing the prepared metal-substituted apatite with hyaluronic acid.

### Experimental Example

### Analysis of metal-substituted apatite

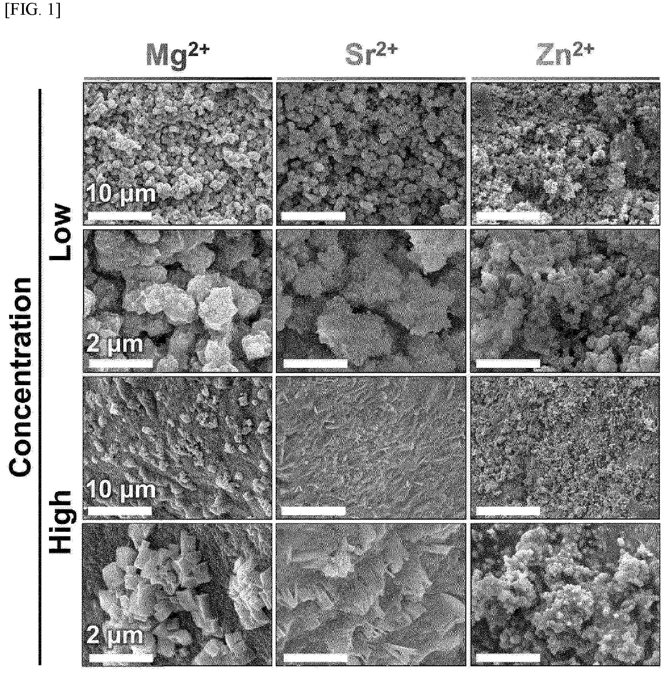

FIG. 1 is an SEM image showing the morphology at low and high amounts of metal when Mg, Sr, and Zn are substituted.

Referring to FIG. 1, it can be confirmed that low-amount substituted apatite was synthesized in a spherical shape, and high-amount substituted apatite was synthesized with a unique morphology for each ion. It can be confirmed that these shapes are ion-substituted apatites obtained when a high content of ions is substituted.

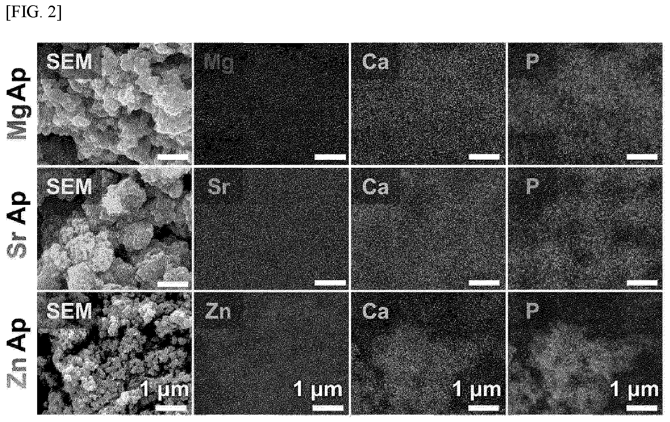

FIG. 2 is an EDS image showing the morphology at low and high amounts of metal when Mg, Sr, and Zn are substituted.

Referring to Figure 2, it can be seen that substituted ions, calcium, and phosphate are all present in the powder, which proves that Mg, Sr, and Zn have actually partially replaced the metal.

### Characterization by content

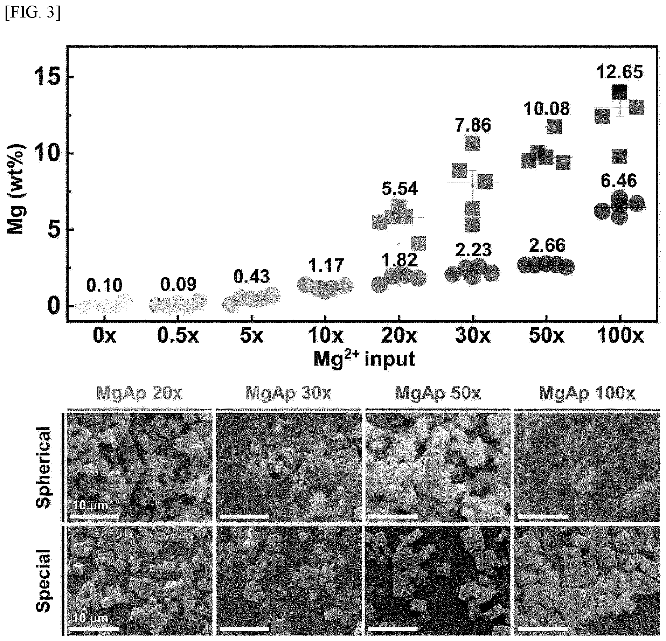

FIG. 3 shows the results of SEM EDX analysis measuring the magnesium content of the composite when synthesized while increasing the amount of Mg added.

In FIG. 3, the 'number x' on the x-axis means 0.5 times the concentration of Mg in human body fluids, and it can be confirmed that this increases in proportion to the amount added when looking at the graph reformulation

In addition, unusually, when Mg ions were added above a certain level, it was confirmed that instead of round particles like in the photos in the top row, quadrangular-shaped particles with greatly increased magnesium content were mixed and synthesized.

The sample with this unique morphology (rectangle) is a material called whitlockite, which is the second most abundant substance among the mineral components of bone, in particular, the present invention utilizes laser hydrothermal synthesis to obtain whitlockite in a very short time.

In addition, the fraction of magnesium in the powder is 0.09 to 12.65 wt%, and the weight percent of magnesium in the powder may range from 0.05 to 20 wt% depending on the concentration.

### Experiment on metal ion elution amount

The metal-substituted apatite powder synthesized according to the present invention was immersed in DMEM, a cell culture medium, maintained at 37 °C for 24 hours, and then filtered through a 0.22µm filter to obtain only the culture medium.

It was thought that metal ions would be eluted within 24 hours, at this time, the degree of ion elution for each material was predicted from the previously obtained icp data, and the ion concentration that may provide good efficacy to osteoblasts was investigated and the powder amount was adjusted according to the corresponding concentration.

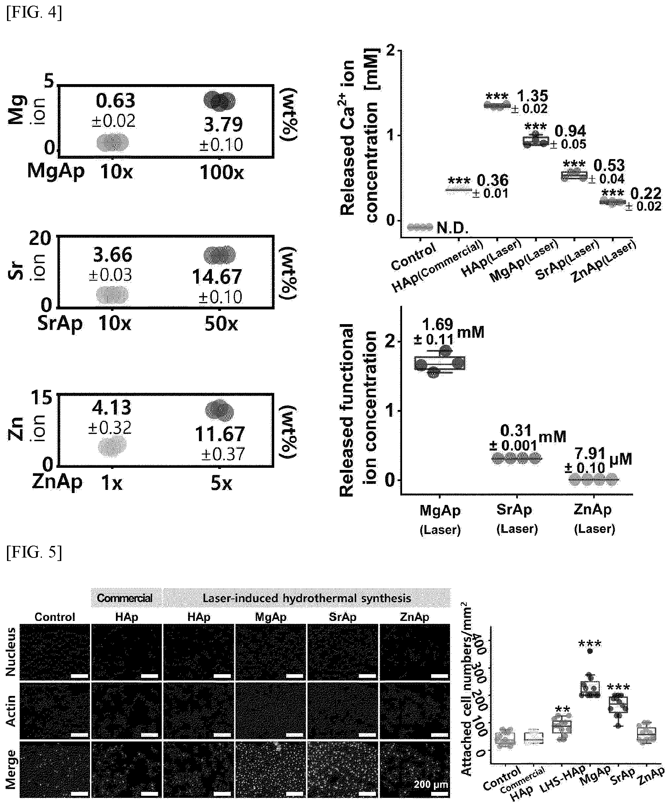

The concentration of the cell culture medium including the ions produced in this way is shown in FIG. 4.

In FIG. 4, the experimental groups are defined as a control group without any substances dissolved, commercial HAP (unsubstituted HAP) taken as a comparison group, HAP obtained through laser hydrothermal synthesis, and high-content MgAp, SrAp, and ZnAp, and additionally, it can be confirmed that the Sr and Zn fractions may also be controlled from 0.5 to 20 wt%.

Referring to FIG. 4, laser-synthesized HAP eluted more than 4 times more Ca ions than commercial HAP, and MgAp and SrAp also eluted a larger amount of Ca ions than commercial HAP.

In addition, in the case of the metal-substituted apatites according to the present invention, substituted metal ions were eluted, i.e., MgAp eluted 1.69 mM of magnesium ions, SrAp eluted 0.31 mM of strontium ions, and ZnAp eluted 7.91 µM of zinc ions. 10% FBS and 1% antibiotics were added to the cell culture medium containing each of these ions, and MC3T3-E1 osteoblasts were cultured. Osteoblasts cultured for 3 hours were utilized to evaluate adhesion, and osteoblasts cultured for 72 hours were utilized to evaluate proliferation.

### Assessment of osteoblast proliferation

FIG. 5 shows an image of the shape of osteoblasts attached after cell culture.

Referring to FIG. 5, while the control group and the commercial HAP group showed a similar number of attached cells, the laser-synthesized HAP, MgAp, and SrAp groups showed a relatively large number of attached cells. In particular, in the case of MgAp, the number of cells increased more than 5-fold in just 3 hours.

### Antibacterial experiment

A medium for the antibiotic ampicillin (AMP) resistance test was prepared in advance, plated onto a plate, and completely hardened to prepare a standard agar medium. The test was evaluated using two forms of samples: samples in the form of powder and samples in the shape of disks. In the case of the test material in powder form, it was placed on a standard agar medium and 100 µL of phosphate buffered saline (PBS) was added and moistened the test material to realize the release and diffusion of ions.

On the other hand, in the case of the disk-shaped test material, a disk pellet with a diameter of 8 mm prepared using a powder molding press (# SERVO PRESS controller) with the test material in powder form was placed on a standard agar medium.

In both types of experiments, a spore suspension of *Escherichia coli* (*E.coli*) transformed with a pUC19 plasmid (induced to be resistant to AMP) was inoculated and sprayed evenly on the sample and medium, placed in an incubator with a temperature of 28 °C to 30°C and a relative humidity of 85% or more, and the degree of growth of *E. coli* during 24 hours was visually observed in the concentric area of the inhibition zone, the diameter was measured, and the results were read.
- SERVO PRESS controller loading conditions: 19.514(MPa), speed: 2.0 (P/S)
- Inoculant bacterial concentration (CFU/mL): E.coli, 1.0 × 10⁶
- Sample: 100 mg/ Φ8mm per section

The results of evaluating the antibacterial activity of the evaluated Mg and Zn ion-substituted apatite (MgAP, ZnAP) specimens are as follows.

**[Table 1]**

| Form | Powder | | Pellet | |
|---|---|---|---|---|
| | Inhibition zone (ϕ, min) | Fold change | Inhibition zone (ϕ, min) | Fold change |
| Negative Cont. (Amp) | 0 | 1 | 0 | 1 |
| Negative Cont. (HAP) | 0 | 1 | 0 | 1 |
| MgAP | 10.4 | 1.3 | 11 | 1.37 |
| ZnAP | 14 | 1.75 | 15 | 1.87 |
| Positive Cont. (Kan) | 40 | 5 | 23 | 2.87 |

Referring to the above results, the substituted test specimen showed excellent bacterial inhibition against transformed antibiotic-resistant *E. coli* after 24 hours, regardless of the specimen's form. On the other hand, hydroxyapatite (HAP) before substitution was observed to be inadequate for inhibiting the growth of bacteria.

The red color visible to the naked eye on the medium is presumed to show the release behavior of ions released from substituted apatite, and is believed to be the main reason for the local antibacterial inhibitory effect. As the growth of *E. coli* was observed to be inhibited in both powder and disk forms, it can be seen that bacterial inhibition may be controlled by the degree of release of substituted ions rather than the formulation.

The filler composition described above may be mixed with hyaluronic acid, and in this case, the apatite powder may be present in an amount of 1 to 50 wt% of the total weight. When the amount of the powder is lower than the above range, it is difficult to expect a sufficient antibacterial effect, and when the amount exceeds the above range, problems such as injection may occur.

### Preparation of filler composition

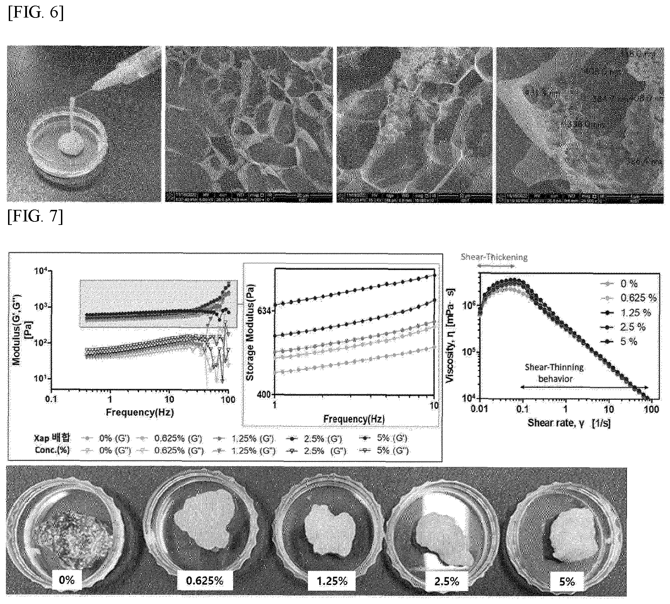

FIG. 6 shows an optical image of a filler composition containing metal-substituted apatite (MgAp) mixed with hyaluronic acid hydrogel being discharged from a syringe according to the present invention, and an SEM image showing the morphology of the composition.

Referring to FIG. 6, the metal-substituted apatite powder was physically mixed with the hyaluronic acid hydrogel, and it can be confirmed that the metal-substituted apatite particles were well mixed within the hyaluronic acid polymer chain. Here, the needle size was 27G x ½in (1.3mm), and regarding the extent to which the contents can be discharged, the apatite powder may be discharged up to 5 wt% of the total weight of hyaluronic acid.

### Rheological property evaluation

FIG. 7 shows the results of measuring viscoelasticity changes and viscosity rheological properties of the composition.

Referring to FIG. 7, as a result of measuring a composition in which metal-substituted apatite powder was mixed at up to 5 wt% of the total weight of the hyaluronic acid hydrogel, it can be confirmed that as the amount of metal-substituted apatite increases, the viscoelasticity increases and the properties of a hydrogel are exhibited. That is, to resist stress caused by various movements, the formulation injected into oral soft tissue requires higher viscoelasticity than existing hyaluronic acid, and therefore, the composition according to the present invention is very advantageous in this respect.

In addition, it was observed that as the shear rate increases, the shape of the gel is maintained while showing shear-thickening behavior in which viscosity increases along with an increase in the flow resistance of the composition, and as the shear rate continued to increase, it shows shear-thinning behavior where viscosity decreases while having the properties of a fluid. This confirms that the formulation of the present invention may be utilized as an injection.

### Evaluation of swelling degree

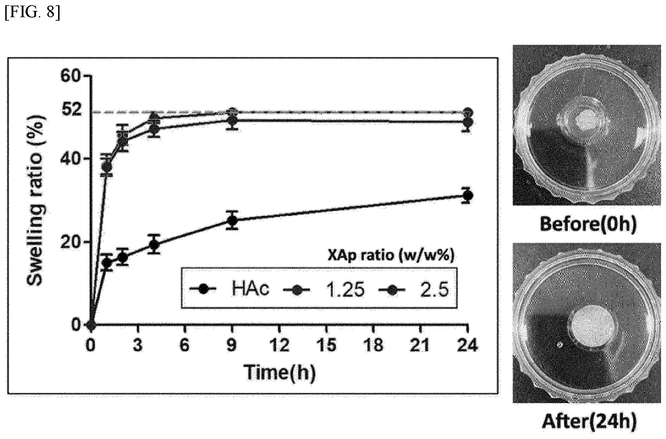

FIG. 8 shows a graph measuring the swelling ratio of the composition, here, the composition was placed on a semi-permeable membrane of a 24-well insert, and the absorption of surrounding phosphate buffered saline (PBS) and swelling degree of the composition were confirmed.

Referring to FIG. 8, it can be seen that the volume and swelling of the composition of the present invention including apatite compared to hyaluronic acid increase over time. These results show swelling even inside the gums, which are always in a humid environment, which can have a positive effect on adhesion to tissue and periodontium restoration.

### Metal ion elution amount experiment for metal-substituted apatite and hydrogel composition

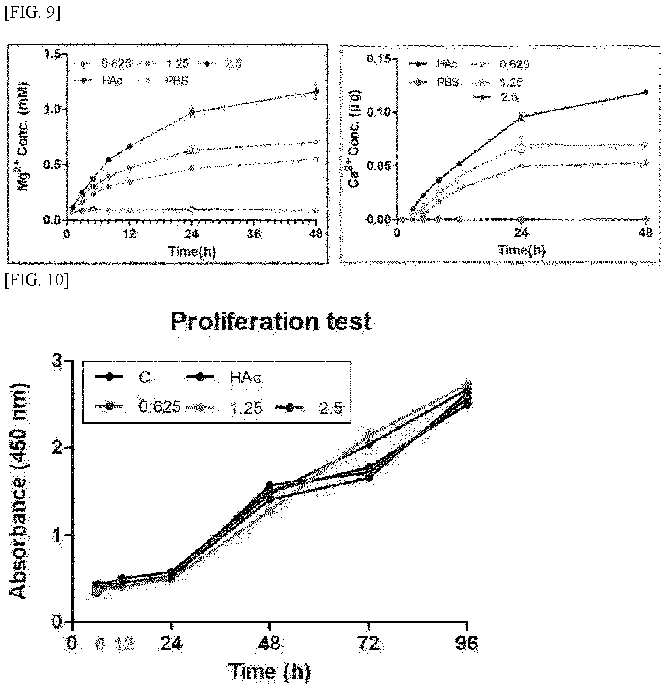

FIG. 9 shows the results of an ion elution amount experiment over time using the composition in phosphate buffered saline (PBS), here, the composition was immersed in PBS, and the amount of metal ions eluted over time was measured at 37 °C for up to 48 hours. Substituted metal apatite was mixed at 0.625, 1.25, and 2.5 wt% of the total hyaluronic acid weight, and commercial hyaluronic acid hydrogel (HAc) was used as a comparison group.

Referring to FIG. 9, while no metal ions were confirmed to be eluted from the hyaluronic acid hydrogel, Mg ions and Ca ions decomposed from the composition containing metal-substituted apatite were eluted over time. Gingival fibroblasts human gingival fibroblast-1 (HGF-1) were cultured by adding 10% FBS and 1% antibiotics to the cell culture medium containing each of these ions. Gingival fibroblasts cultured for 96 hours were utilized to evaluate proliferation.

### Evaluation of gingival fibroblast proliferation

FIG. 10 shows the results of CCK-8 analysis conducted to determine changes in cell proliferation according to metal ions eluted after cell culture.

Referring to FIG. 10, the eluted metal ions, Mg and Ca ions, did not show a significant effect on gingival fibroblast proliferation.

### Effect of metal-substituted apatite eluates on the expression of collagen type I at mRNA level.

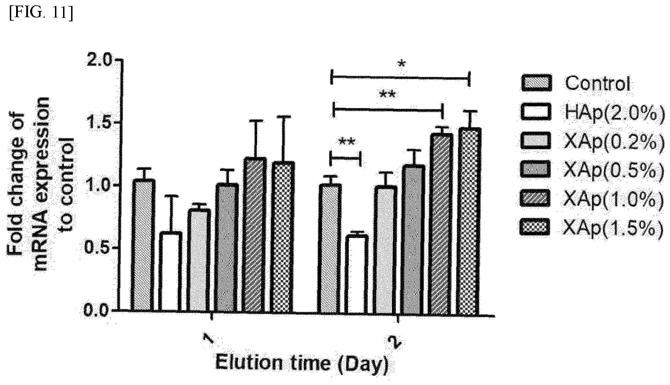

FIG. 11 is a result showing that the metal ions of the present invention increase collagen expression in gingival fibroblasts, here, metal-substituted apatite was maintained in a cell culture medium including 10% FBS and 1% antibiotics at 37 °C for 24 hours (1 day) and 48 hours (2 days), and then filtered through a 0.22 µm filter to obtain only the culture fluid, which was defined as the eluate. At this time, metal-substituted apatite was eluted by measuring powder amounts of 0.2, 0.5, 1.0, and 1.5 wt% relative to the total weight of hyaluronic acid, and as a comparison group, 2.0 wt% of apatite (HAp) synthesized by laser hydrothermal synthesis was measured and eluted. After the elution time eluate was treated with gingival fibroblasts at 37 °C for one day, the mRNA expression level of collagen type I alpha 1 (Col1a1) was quantified by quantitative real-time RT-PCR. Collagen type I is abundant in the dermis and is involved in skin tissue recovery. *P < 0.05, **P < 0.01. Error bars represent the standard deviation of four replicate samples from a single representative experiment.

Referring to FIG. 11, collagen expression was decreased in the comparison group. On the other hand, in the experimental group in which metal-substituted apatite was eluted, a significant increase was confirmed in the culture treatment group in which the metal-substituted apatite was eluted at concentrations of 1.0 wt% and 1.5 wt% for 48 hours. The above results suggest that metal ions eluted from metal-substituted apatite promote collagen mRNA expression in gingival fibroblasts. When the metal-substituted apatite is included within the above range in hyaluronic acid, there is an advantage of showing a long-term tissue recovery effect due to the effect of metal ions continuously eluted as a sustained-release composition.

## Claims

1. An apatite for a filler, comprising an apatite that contains metal, wherein the metal entirely or partially replaces the calcium of the apatite.

2. The apatite of claim 1, wherein the metal is at least one selected from the group consisting of magnesium (Mg), zinc (Zn), and strontium (Sr).

3. The apatite of claim 2, wherein the metal of the apatite replaces the calcium of the apatite in a solution using a laser.

4. The apatite of claim 3, wherein the apatite has a quadrangular shape.

5. A filler composition comprising:
the apatite according to any one of claims 1 to 4; and
hyaluronic acid.

6. The filler composition of claim 5, wherein the metal is eluted in the form of ions in vivo.

7. The filler composition of claim 6, wherein the filler composition is for oral tissue.

8. The filler composition of claim 7, wherein the filler composition has antibacterial properties as it is eluted in the form of ions in vivo.

9. The filler composition of claim 1, wherein in the apatite containing the metal, the metal is present in an amount of 0.05 to 20% by weight of the total apatite.

10. The filler composition of claim 5, wherein the apatite is present in an amount of 1 to 50% by weight of the composition.

11. The filler composition of claim 6, wherein the metal ions include magnesium ions.

12. The filler composition of claim 11, wherein the composition swells in body fluid.
